(19) Europäisches Patentamt
European Patent Office
Office européen des brevets

(11) Veröffentlichungsnummer: **0 043 798**
**A1**

# (12) EUROPÄISCHE PATENTANMELDUNG

(21) Anmeldenummer: **81810260.0**

(22) Anmeldetag: **22.06.81**

(51) Int. Cl.³: **C 07 D 231/20**, A 01 N 43/18

(30) Priorität: **27.06.80 CH 4968/80**
**29.12.80 CH 9609/80**

(43) Veröffentlichungstag der Anmeldung: **13.01.82**
**Patentblatt 82/2**

(84) Benannte Vertragsstaaten: **AT BE CH DE FR GB IT LI NL**

(71) Anmelder: **CIBA-GEIGY AG, Patentabteilung Postfach, CH-4002 Basel (CH)**

(72) Erfinder: **Gsell, Laurenz, Dr., Maiengasse 56, CH-4056 Basel (CH)**
Erfinder: **Gehret, Jean-Claude, Dr., Im Aeschfeld 12, CH-4147 Aesch (CH)**

(54) **Pyrazolylcarbamate und ihre Verwendung als Pestizide.**

(57) Pyrazolylcarbamate der Formel

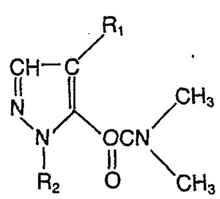

worin $R_1$ Wasserstoff oder $C_2$-$C_5$-Alkyl und $R_2$ $C_1$-$C_7$-Alkyl, gegebenenfalls substituiert, $C_2$-$C_5$-Alkenyl oder $C_2$-$C_5$-Alkinyl bedeuten. Ein Verfahren zur Herstellung dieser Pyrazolylcarbamate und ihre Verwendung in der Schädlingsbekämpfung sind beschrieben.

EP 0 043 798 A1

CIBA-GEIGY AG
Basel (Schweiz) 5-12936/1+2/=

BEZEICHNUNG GEÄNDERT
siehe Titelseite

Neue Pyrazolyle

Die vorliegende Erfindung betrifft Pyrazolylcarbamate, Verfahren zu ihrer Herstellung und ihre Verwendung in der Schädlingsbekämpfung.

Die Pyrazolylcarbamate haben die Formel

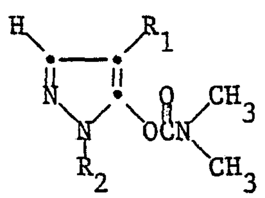

(I)

worin $R_1$ Wasserstoff oder $C_1$-$C_5$-Alkyl und $R_2$ $C_1$-$C_7$-Alkyl, gegebenenfalls substituiert, $C_2$-$C_5$-Alkenyl oder $C_2$-$C_5$-Alkinyl bedeuten.

Die für $R_1$ und $R_2$ in Frage kommenden Alkyl-, Alkenyl- und Alkinylgruppen können geradkettig oder verzweigt sowie im Falle der Alkylgruppen für $R_2$ auch durch Halogen, insbesondere Chlor, Cyano, Hydroxy, $C_1$-$C_5$-Alkoxy, $C_1$-$C_5$-Alkylthio oder $C_1$-$C_5$-Alkyloxycarbonyl substituiert sein. Beispiele solcher Gruppen sind u.a.: Methyl, Aethyl, Methoxyäthyl, Methylthioäthyl, Cyanoäthyl, Chloräthyl, Hydroxyäthyl, Propyl, Isopropyl, n-Butyl, n-Pentyl, n-Hexyl, n-Heptyl und deren Isomere, Allyl, Propargyl.

Wegen ihrer Wirkung bevorzugt sind Verbindungen der Formel I, worin $R_1$ Methyl und $R_2$ $C_1$-$C_4$-Alkyl, Chloräthyl, Cyanoäthyl, Hydroxyäthyl, Methoxyäthyl, Methylthioäthyl, Allyl oder Propargyl bedeuten.

Insbesondere bevorzugt sind Verbindungen der Formel I, worin $R_1$ Methyl und $R_2$ Aethyl oder Isopropyl bedeuten.

Die Verbindungen der Formel I können nach an sich bekannten Methoden, z.B. wie folgt hergestellt werden:

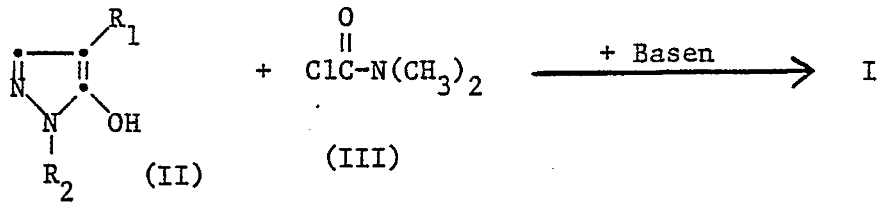


(II) (III)

In der Formel II haben $R_1$ und $R_2$ die für die Formel I angegebene Bedeutung. Als geeignete Basen kommen insbesondere tertiäre Amine wie Triäthylamine, Dialkylaniline und p-Dialkylaminopyridine in Betracht.

Das Verfahren wird in der Regel bei normalem Druck, bei einer Temperatur von -25° bis 150°C, vorzugsweise bei 50-100°C, und in einem gegenüber den Reaktionsteilnehmern inerten Lösungs- oder Verdünnungsmitteln durchgeführt.

Als Lösungs- oder Verdünnungsmittel eignen sich z.B. Aether und ätherartige Verbindungen, wie Diäthyläther, Dipropyläther, Dioxan, Dimethoxyäthan und Tetrahydrofuran; Amide wie N,N-dialkylierte Carbonsäureamide; aliphatische, aromatische sowie halogenierte Kohlenwasserstoffe, insbesondere Benzol, Xylole, Chloroform und Chlorbenzol; Nitrile wie Acetonitrile; Dimethylsulfoxid und Ketone, wie Aceton und Methyläthylketon.

Die Ausgangsstoffe der Formeln II und III sind bekannt oder können nach bekannten Methoden hergestellt werden.

Die Verbindungen der Formel I eignen sich zur Bekämpfung von verschiedenartigen Schädlingen an Tieren und Pflanzen.

Insbesondere eignen sich die Verbindungen der Formel I zur Bekämpfung aller Entwicklungsstadien von Insekten, phytopathogenen Milben sowie von Zecken, z.B. der Ordnungen Lepidoptera, Coleoptera, Homoptera,

Heteroptera, Diptera, Acarina, Thysanoptera, Orthoptera, Anoplura, Siphonaptera, Mallophaga, Thysanura, Isoptera, Psocoptera und Hymenoptera.

Vor allem eignen sich Verbindungen der Formel I zur Bekämpfung von pflanzenschädigenden Insekten, insbesondere pflanzenschädigenden Frassinsekten, in Zier- und Nutzpflanzen, insbesondere in Baumwollkulturen (z.B. gegen Spodoptera littoralis und Heliothis virescens) und Gemüsekulturen (z.B. gegen Leptinotarsa decemlineata und Myzus persicae).

Wirkstoffe der Formel I zeigen auch eine sehr günstige Wirkung gegen Fliegen, wie z.B. Musca domestica und Mückenlarven. Daneben zeichnen sich die Verbindungen der Formel I durch eine breite ovizide und ovilarvizide Wirkung aus.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie

z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxyd-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im(aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglycoläther, Polypropylen-Polyäthylenoxydaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgender Publikation beschrieben:

"Mc Cutcheon's Detergents and Emulsifiers Annual" MC Publishing Corp., Ringwood, New Jersey, 1979.

Die pestiziden Zubereitungen enthalten in der Regel 0,1 bis 99%, insbesondere 0,1 bis 95%, Wirkstoff der Formel I, 1 bis 99,9% eines festen oder flüssigen Zusatzstoffes und 0 bis 25%, insbesondere 0,1 bis 25%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Formulierungsbeispiele für flüssige Wirkstoffe der Formel I

(% = Gewichtsprozent)

| 1. Emulsions-Konzentrate | a) | b) | c) |
|---|---|---|---|
| Wirkstoff | 20% | 40% | 50% |
| Ca-Dodecylbenzolsulfonat | 5% | 8% | 5,8% |
| Ricinusöl-polyäthylenglykoläther (36 Mol AeO) | 5% | - | - |
| Tributylphenoyl-polyäthylenglykoläther (30 Mol AeO) | - | 12% | 4,2% |
| Cyclohexanon | - | 15% | 20% |
| Xylolgemisch | 70% | 25% | 20% |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff | 80% | 10% | 5% | 95% |
| Aethylenglykol-monomethyl-äther | 20% | - | - | - |
| Polyäthylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

Die Lösungen sind zur Anwendung in Form kleinster Tropfen geeignet.

| 3. Granulate | a) | b) |
|---|---|---|
| Wirkstoff | 5% | 10% |
| Kaolin | 94% | - |
| Hochdisperse Kieselsäure | 1% | |
| Attapulgit | - | 90% |

Der Wirkstoff wird in Methylenchlorid gelöst, auf den Träger aufgesprüht und das Lösungsmittel anschliessend im Vakuum abgedampft.

| 4. Stäubemittel | a) | b) |
|---|---|---|
| Wirkstoff | 2% | 5% |
| Hochdisperse Kieselsäure | 1% | 5% |
| Talkum | 97% | - |
| Kaolin | - | 90% |

Durch inniges Vermischen der Trägerstoffe mit dem Wirkstoff erhält man gebrauchsfertige Stäubemittel.

Formulierungsbeispiele für feste Wirkstoffe der Formel I

(% = Gewichtsprozent)

| 5. Spritzpulver | a) | b) |
|---|---|---|
| Wirkstoff | 20% | 60% |
| Na-Ligninsulfonat | 5% | 5% |
| Na-Laurylsulfat | 3% | - |
| Na-Diisobutylnaphthalinsulfonat | - | 6% |
| Octylphenolpolyäthylenglykoläther (7-8 Mol AeO) | - | 2% |
| Hochdisperse Kieselsäure | 5% | 27% |
| Kaolin | 67% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

6. Emulsions-Konzentrat

| | |
|---|---|
| Wirkstoff | 10% |
| Octylphenolpolyäthylenglykoläther (4-5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| 7: Stäubemittel | a) | b) |
|---|---|---|
| Wirkstoff | 5% | 8% |
| Talkum | 95% | - |
| Kaolin | - | 92% |

Man erhält anwendungsfertige Stäubemittel, indem der Wirkstoff mit dem Träger vermischt und auf einer geeigneten Mühle vermahlen wird.

8. Extruder Granulat

| | |
|---|---|
| Wirkstoff | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

9. Umhüllungs-Granulat

| | |
|---|---|
| Wirkstoff | 3% |
| Polyäthylenglykol (M G 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

10. Suspensions-Konzentrat

| | |
|---|---|
| Wirkstoff | 40% |
| Aethylenglykol | 10% |
| Nonylphenolpolyäthylenglykoläther ( 15 Mol AeO) | 6% |
| Na-Lingninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Herstellungsbeispiel

2-Aethyl-4-methyl-5-H-pyrazol-3-yl-N,N-dimethylcarbamat

a) Zu 232 g Formylpropionsäuremethylester in 100 ml Methanol werden 118 g Aethylhydrazin in 1000 ml Aethanol und 100 ml Wasser langsam zugetropft. Die exotherme Reaktion wird unter 40°C gehalten und durch anschliessendes Kochen zum Rückfluss während 1 Stunde zu Ende geführt. Nach 12 Stunden bei Raumtemperatur wird das Produkt abfiltriert und mit Alkohol/Aether gewaschen. Man erhält die Verbindung der Formel

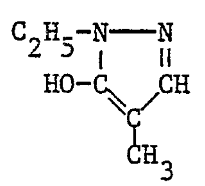

mit einem Schmelzpunkt von 115-117°C. Auf analoge Weise wird auch die Verbindung der Formel

(i) 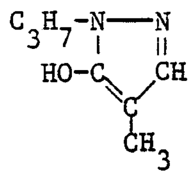 Smp.: 165-168°C

hergestellt.

b) 12,6 g 2-Aethyl-3-hydroxy-4-methyl-5-H-pyrazol, 9,2 ml Dimethylcarbamoylchlorid, 13,8 ml Triäthylamin und 100 mg 4-Dimethyl-aminopyridin in 200 ml Chloroform werden 6 Stunden bei 60°C gehalten. Das Lösungsmittel wird entfernt und der Rückstand zwischen Wasser und Methylenchlorid verteilt, die Methylenchloridphase mit Sole gewaschen, über $Na_2SO_4$ getrocknet und eingeengt. Das Rohprodukt wird an Kieselgel mit Petroläther/Aether 1:1 als Eluiermittel chromatographiert. Man erhält die Verbindung der Formel

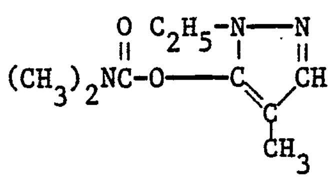

mit einer Refraktion von $n_D^{20°} = 1,4843$.

Auf analoge Weise werden auch die Verbindungen der Formel

(i) 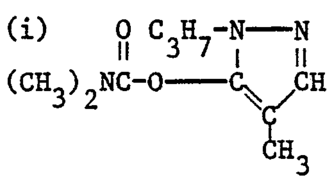 $n_D^{20°} = 1,4809$

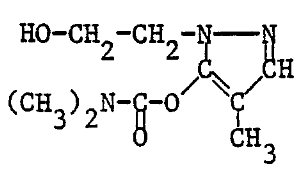 Smp.: 87-89°C

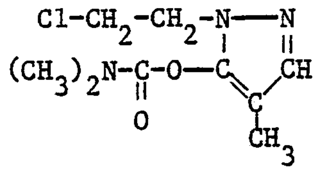

$n_D^{20°} = 1,5009$

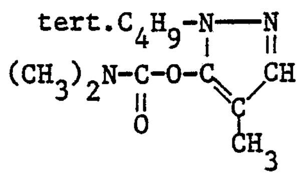

$n_D^{20°} = 1,4810$

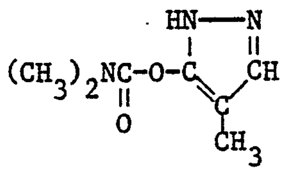

Smp. 87-90°C

hergestellt.

Beispiel 2: Insektizide Kontakt-Wirkung: Aphis craccivora und Myzus persicae.

In Töpfen angezogene Pflanzen (Vicia faba) wurden vor dem Versuchsbeginn je mit ca. 200 Individuen der Spezies Aphis fabae oder Myzus persicae besiedelt. Die so behandelten Pflanzen wurden 24 Stunden später mit einer Lösung enthaltend 200 oder 100 ppm der zu prüfenden Verbindung bis zur Tropfnässe besprüht. Man verwendete pro Test-Verbindung und pro Konzentration zwei Pflanzen und eine Auswertung der erzielten Abtötungsrate erfolgte nach weiteren 24 Stunden.

Testresultate

| | Konzentration in ppm zur 100%igen Abtötung von | |
|---|---|---|
| | Aphis craccivora | Myzus persicae |
| 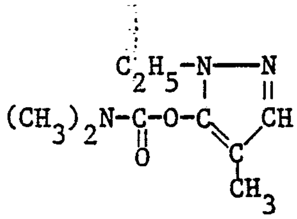 | 100 | 100 |

0043798

| | Konzentration in ppm zur 100%igen Abtötung von | |
|---|---|---|
| | Aphis craccivora | Mycus persicae |
| 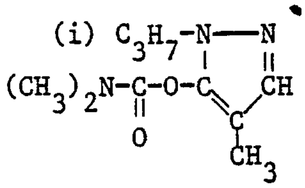 | 100 | 100 |
| 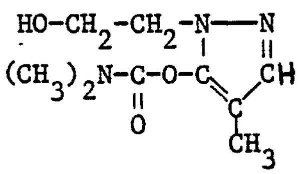 | 100 | 200 |
| 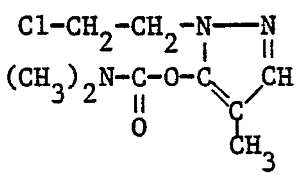 | 100 | 100 |
| 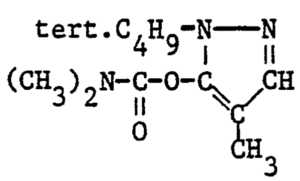 | 200 | 200 |
| 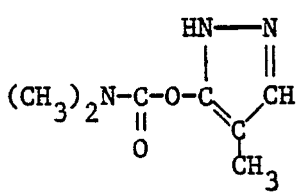 | 100 | 200 |

Beispiel 3: Insektizide Wirkung (systemisch): Aphis craccivora

Bewurzelte Bohnenpflanzen wurden in Töpfe, welche 600 ccm Erde enthalten, verpflanzt und anschliessend 50 ml einer Lösung der zu prüfenden Verbindung in einer Konzentration von 50 ppm oder 10 ppm direkt auf die Erde aufgegossen.

Nach 24 Stunden wurden auf die oberirdischen Pflanzenteile Läuse der Spezies Aphis craccivora gesetzt und die Pflanzen mit einem Plastikzylinder überstülpt, um die Läuse vor einer eventuellen Kontakt- oder Gaswirkung der Testsubstanz zu schützen.

Die Auswertung der erzielten Abtötung erfolgte 48 und 72 Stunden nach Versuchsbeginn. Pro Konzentrationsdosis Testsubstanz wurden zwei Pflanzen, je eine in einem separaten Topf, verwendet. Der Versuch wurde bei 25°C und 70% relativer Luftfeuchtigkeit durchgeführt.

Die Verbindungen gemäss Beispiel 1 zeigten innerhalb der oben angegebenen Konzentrationsgrenzen eine 100%ige Wirkung gegen Insekten der Spezies Aphis craccivora.

Patentansprüche

1. Ein Pyrazolylcarbamat der Formel

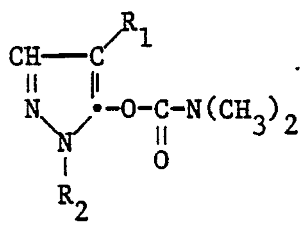

worin $R_1$ Wasserstoff oder $C_1$-$C_5$-Alkyl und $R_2$ $C_1$-$C_7$-Alkyl, gegebenenfalls substituiert, $C_2$-$C_5$-Alkenyl oder $C_2$-$C_5$-Alkinyl bedeuten.

2. Eine Verbindung gemäss Anspruch 1, dadurch gekennzeichnet, dass $R_1$ Methyl und $R_2$ $C_1$-$C_4$-Alkyl, Chloräthyl, Cyanoäthyl, Hydroxyäthyl, Methoxyäthyl, Methylthioäthyl, Allyl oder Propargyl bedeuten.

3. Eine Verbindung gemäss Anspruch 2, dadurch gekennzeichnet, dass $R_1$ Methyl und $R_2$ Aethyl oder Isopropyl bedeuten.

4. Die Verbindung gemäss Anspruch 3 der Formel

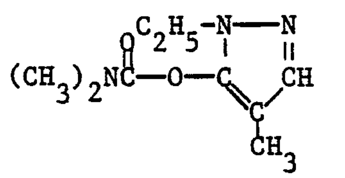

5. Die Verbindung gemäss Anspruch 3 der Formel

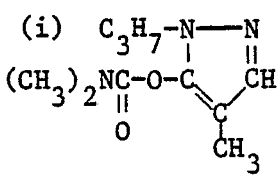

6. Ein Verfahren zur Herstellung von Verbindungen der Formel I, dadurch gekennzeichnet, dass man in Gegenwart einer Base eine Verbindung der Formel

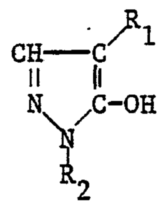

,

worin $R_1$ und $R_2$ die im Anspruch 1 angegebene Bedeutung haben, mit der Verbindung der Formel

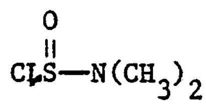

umsetzt.

7. Schädlingsbekämpfungsmittel, welches neben geeigneten Formulierungshilfsmittel als aktive Komponente eine Verbindung gemäss Anspruch 1 enthält.

8. Verwendung einer Verbindung gemäss Anspruch 1 zur Bekämpfung von Schädlingen an Tieren und Pflanzen.

9. Verwendung gemäss Anspruch 8 zur Bekämpfung von Insekten und Vertretern der Ordnung Akarina.

0043798

Nummer der Anmeldung

Europäisches Patentamt

# EUROPÄISCHER RECHERCHENBERICHT

EP 81 81 0260

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | DE - A - 2 644 589 (BAYER A.G.)<br>* Patentansprüche *<br>-- | 1,6-9 | C 07 D 231/20<br>A 01 N 43/18 |
| | US - A - 2 681 915 (H. GYSIN et al)<br>* Spalte 1 *<br>-- | 1,6-9 | |
| | CH - A - 282 655 (J.R. GEIGY A.G.)<br>* Beispiele *<br>-- | 1,6-9 | **RECHERCHIERTE SACHGEBIETE (Int. Cl.³)** |
| P | EP - A - 0 023 326 (BAYER A.G.)<br>* Seiten 1-2 *<br>---------- | 1,6-9 | C 07 D 231/20<br>A 01 N 47/18 |

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 16-09-1981 | BRIGHENTI |